# EUROPEAN PATENT SPECIFICATION

(11) **EP 2 316 380 B1**
(45) Date of publication and mention of the grant of the patent: **11.07.2018**
(21) Application number: 10251874.3
(22) Date of filing: 29.10.2010
(51) Int. Cl.: A61F 2/08

(54) **Dual cannula system for partial thickness rotator cuff repair**
Zweikanülensystem zur Reparatur der partiellen Rotatorenmanschette
Système à double canule pour la réparation de la coiffe du rotateur à épaisseur partielle

(30) Priority: 30.10.2009 US 609147
(43) Date of publication of application: 04.05.2011
(73) Proprietor: DePuy Mitek, Inc., Raynham, MA 02767 (US)
(72) Inventor: Whittaker, Gregory R., Stoneham, MA 02180 (US); Sengun, Mehmet Ziya, Canton, MA 02021 (US); Dimatteo, Kristian, Waltham, MA 02451 (US)
(74) Representative: Tunstall, Christopher Stephen

(56) References cited:
- EP-A1- 1 884 199
- US-A- 5 840 078
- US-B1- 6 206 886

## Description

### Background

The present disclosure relates to systems for performing a repair of a partial thickness rotator cuff tear.

A PASTA (partial articular surface tendon avulsion) lesion in a rotator cuff of a shoulder can be particularly difficult to repair. The rotator cuff comprises a group of muscles which surround the shoulder and tendons which attach those muscles to the humeral head. The tendons have a footprint where they attach to the humeral head and in a PASTA lesion a portion of the tendon's footprint becomes detached from the humeral head. Such lesions are most commonly found on the supraspinatus tendon.

One option for treatment is completion of the tear and repair using standard techniques for a full thickness tear. Preservation of the existing attachment is thus lost and the entire tendon must be reattached. Another option comprises screwing a threaded suture anchor through the tendon and into the humeral head, passing suture through the tendon and tying down the tendon to effect reattachment. This causes further trauma to the tendon.

US 5840078 discusses a method and apparatus for mechanical attachment of soft tissue to bone tissue, including a metallic cannulated rivet adapted for implantation in a bone mass.

### Summary of the Invention

Claim 1 defines the invention and the dependent claims disclose the preferred embodiments. The present disclosure provides systems for repairing a PASTA lesion which provides advantages over current treatment options by minimizing trauma to the tendon as a suture anchor is being passed therethrough. A trans-soft tissue anchor implantation system according to the present invention comprises a positioning wire, a cannula system and a suture anchor. The positioning wire has a tissue penetrating distal tip. The cannula system for passage through the soft tissue comprises an inner cannula having a sharp distal tip, an axial lumen therethrough sized to accommodate the positioning wire and a proximal end; and an outer cannula having a distal end, a proximal end and an axial lumen therethrough sized to accommodate the inner cannula and coaxially receiving the inner cannula. The outer cannula distal end is tapered to present a gradually increasing profile and the distal tip of the inner cannula
extends distally beyond the end of the distal end of the outer cannula so that as the cannula system is passed through the tissue it more gently dilates and expands an opening therethrough. The suture anchor is sized to fit through the outer cannula lumen. The suture anchor preferably has a length of suture attached, thereto.

Preferably, the positioning wire comprises a textured outer surface.

Preferably, the outer cannula and inner cannula engage to prevent them from sliding apart. In one aspect of the invention the engagement is a frictional.

Preferably, the outer cannula carries depth indicia.

The system is provided with exemplary instructions for use which include the steps of: locating a desired anchor receiving site on the bone; passing the locating wire through the soft tissue and onto or into the bone at the anchor receiving site; passing the cannula system over the locating wire; removing the inner cannula and the locating wire; and passing the suture anchor through the outer cannula and driving the suture anchor into the bone at the anchor site.

An exemplary method according to the present disclosure provides for passing a suture anchor through a soft tissue and into a bone. The method comprises the steps of: locating a desired anchor receiving site on the bone; passing a locating wire through the soft tissue and onto or into the bone at the anchor receiving site; passing over the locating wire an inner/outer cannula system which comprises: an inner cannula having a tapered, sharp distal tip, and an axial lumen therethrough sized to accommodate the positioning wire; and an outer cannula having a distal end, and an axial lumen therethrough sized to accommodate the inner cannula and coaxially receiving the inner cannula, the distal end being tapered wherein to present a gradually increasing profile and wherein the distal tip of the inner cannula extends distally beyond the end of the distal end of the outer cannula; passing the sharp distal tip of the inner cannula through the soft tissue to create an opening therethrough; passing the tapered distal end of the outer cannula through the opening to expand the opening and minimize removal, cutting and disturbance of the tissue as it passes therethrough; removing the inner cannula and the locating wire; and passing the suture anchor through the outer cannula and driving the suture anchor into the bone at the anchor site.

Preferably, the inner cannula and the outer cannula are fixed together during the steps of passing them through the soft tissue. Preferably one or more sutures, or limbs of a single suture, are passed from the suture anchor through the soft tissue. For instance a pair of suture limbs from the suture anchor can be passed through the soft tissue at two different locations and then attaching them together to hold the soft tissue against the bone.

In one aspect of the disclosure, the soft tissue comprises a tendon, such as a rotator cuff tendon having a PASTA lesion.

### Brief Description of the Drawings

FIG. 1 is a perspective view of a suture anchor;
FIG. 2 is a side elevation view of the suture anchor of FIG. 1 loaded onto a driver;
FIG. 3 is a top plan view of the suture anchor of FIG. 1;
FIG. 4. is a side elevation view of a humerus and associated rotator cuff tendon suffering a PASTA lesion showing a K wire being inserted through the tendon to a desired location for placing a suture anchor;
FIG. 5. is a side elevation view of the tendon of FIG. 4 showing a cannula system being passed through the tendon over the K wire;
FIG. 6 is a perspective view of the cannula system of FIG. 5;
FIG. 7 is a side elevation view of the tendon of FIG. 4 a suture anchor loaded onto a driver being passed therethrough via an outer portion of the cannula system;
FIG. 8 is a side elevation view of the tendon of FIG. 4 showing the suture anchor implanted into the humerus beneath the tendon and a limb of suture passing from the suture anchor out of an anterior cannula;
FIG. 9 is a side elevation of the tendon of FIG. 4 showing a spinal needle passed through a location on the tendon and a suture retriever being passed through the spinal needle and out of the anterior cannula;
FIG. 10 is a side elevation of the tendon of FIG. 4 showing both suture limbs passed from the suture anchor and through the tendon at different locations; and
FIG. 11 is a side elevation of the tendon of FIG. 4 showing the suture limbs knotted together to compress the tendon to the humerus thus effecting repair of the PASTA lesion.

### Detailed Description

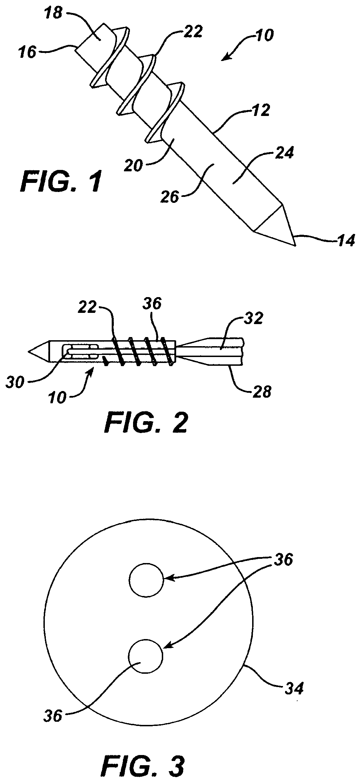

FIG. 1 depicts a suture anchor 10. It comprises an elongated body 12 having a pointed distal tip 14 and a proximal end 16. An axial passageway 18 extends into the body 12 from the proximal end 16. The passageway 18 is open along its sides 20. A thread 22 encircles the body 12. A suture bridge 24 spans the passageway 18 laterally at a distal portion 26 thereof.

Turning also now to FIGS. 2 and 3, an inserter 28 fits into the passageway 18. A length of suture 30 passes around the suture bridge 24 and is received within longitudinal grooves 32 on the inserter 28. As best seen in FIG. 3, the cross-sectional shape of the passageway 18 at the proximal end 16 is essentially a hexagon 34 with a pair of suture passages 36 on opposite corners thereof. The suture passages 36 lead to either side of the suture bridge 24. The inserter 28 has a complimentary shape to fit within the hexagon 34 with its grooves 32 in alignment with the suture passages 36 on the anchor 10.

The suture anchor 10 as shown with the suture passages 36 penetrating the body 12 to leave the passageway 18 open except for the thread 22 minimizes its cross section to provide the least trauma to soft tissue through which it will pass while still having sufficient mechanical strength for the driver 28 to drive it into bone. Where additional fixation strength within the bone may be required the cross section of the anchor 10 could be enlarged, in which case the suture passages 36 need then not necessarily penetrate the body 12 laterally. The anchor 10 can be formed of any suitable biocompatible material such as stainless steel, titanium, cobalt chrome, PEEK (polyaryletheretherketone), other biocompatible polymers, polymer-ceramic composites, bioabsorbable polymers and the like.

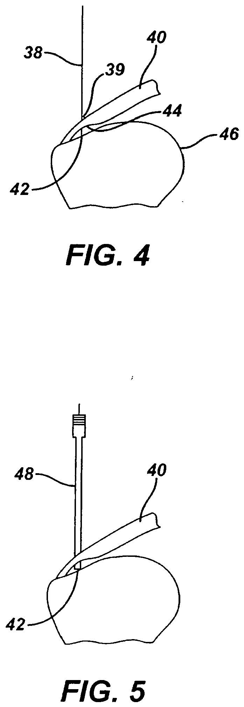

FIGS. 4 to 10 illustrate a procedure to repair a PASTA lesion using the suture anchor 10 of FIG. 1. As seen in FIG. 4, either percutaneously or arthroscopically, a Kirschner wire (K wire) 38 is inserted at a first location 39 through a tendon 40 of a rotator cuff to a desired anchor site 42 beneath its attachment footprint 44 and positioned upon an associated humeral head 46. The K wire 38 can be tapped in or merely positioned at the site 42. To ease manipulation of the K wire 38 it is preferably textured on its outer surface and may be provided with a removable proximal handle (not shown). This site 42 on the humeral head 46 is where the suture anchor 10 (see FIG. 1) will be implanted.

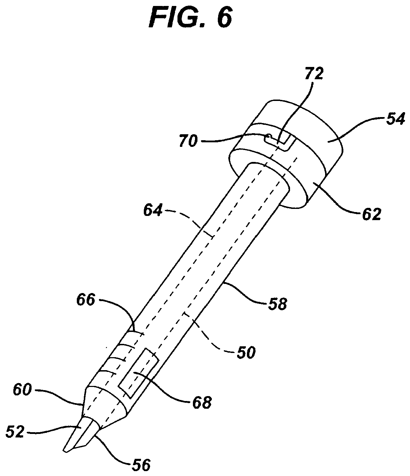

As seen in FIG. 5, a cannula system 48 is passed over the K wire 38 and through the tendon 40 to the site 42. FIG. 6 shows the cannula 48 in more detail. It comprises an inner cannula 50 having a sharp distal tip 52, proximal handle 54 and a lumen 56 therethrough. The inner cannula 50 fits within an outer cannula 58 which has a distal end 60, proximal handle 62 and lumen 64 therethrough. The distal tip 52 of the inner cannula 50 extends slightly beyond the distal end 60 of the outer cannula 58 and the distal end 60 is tapered so that rather than core through the tendon 40 the distal tip 52 creates a small hole and the tapering on the distal tip 52 and distal end 60 allow the cannula system 48 to push aside the tissue and create the smallest hole through the tendon 40 with the least damage thereto. Prior cannulas were inserted through a slit cut into the tissue. The cannula system 48 dilates the tissue gently to minimize trauma to the tissue. The outer cannula 58 has lines 66 which provide a visual indication of depth penetration and also a visualization window 68 which aids in anchor insertion and assessment of appropriate depth into the bone. To prevent slippage of the inner cannula 50 relative to the outer cannula 58 during insertion so provision is preferably provided to help keep them together. Shown are an interlocking nub 70 and groove 72, but other options such as a friction fit, threading , magnets etc. could be employed.

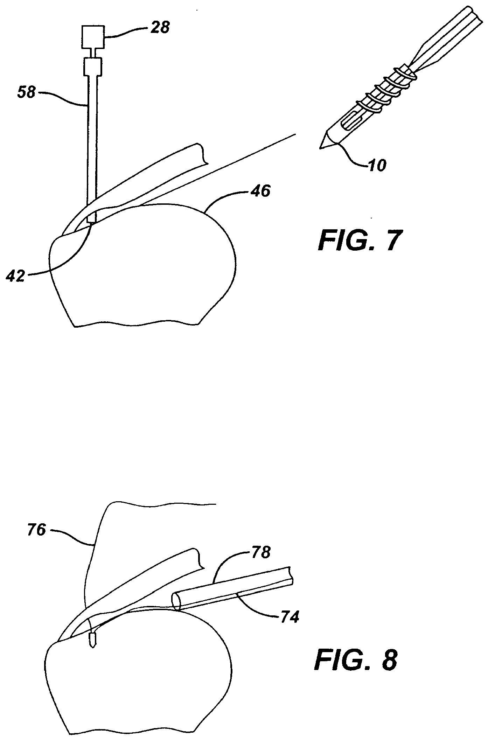

As seen in FIG. 7, in preparation for insertion of the anchor 10, the K wire 38 and inner cannula 50 are removed leaving the outer cannula 58 positioned at the anchor site 42. The suture anchor 10 is preloaded onto the inserter 28, with the suture 30 in place around the suture bridge 24 and passing through the suture passages 36 and grooves 32 (see FIG. 2), is passed down through the outer cannula lumen 60 to the anchor site 42 and is then driven into the humeral head 46. If the anchor 10 is formed of a biocompatible metal such as stainless steel or titanium it can be simply twisted in via the inserter 28. If instead it is formed of a bioabsorbable polymer or other material having less strength a pilot hole should be prepared such as with a drill, tap or awl, at the site 42 through the cannula 46 prior to inserting the anchor 10 through the lumen 60. The inserter 28 and outer cannula 58 can then be removed leaving first and second suture limbs, 74 and 76 respectively, passed up through the tendon 40 at the first location 39 through which the cannula 48 had passed. As seen in FIG. 8, the first suture limb 74 is then retrieved through an auxiliary cannula 78 such as via a grasper (not shown).

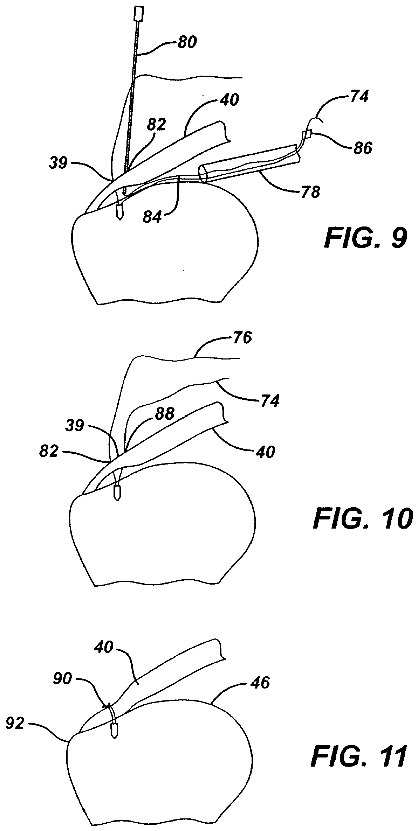

As seen in FIG. 9 a spinal needle 80 is passed through the tendon 40 at a second location 82 spaced apart from the first location 39. A flexible wire suture capture device 84 having a suture capture loop 86 (such as a Chia Percpasser available from DePuy Mitek, Inc. of Raynham, MA) is passed through the spinal needle 80 and retrieved out through the auxiliary cannula 78 so that the first suture limb 74 can be threaded through the suture capture loop 86. When the spinal needle 80 and suture capture device 84 are pulled back through the skin this pulls the first suture limb 74 through the tendon 40 at the second location 82. For a quick procedure, the first and second suture limbs 74 and 76 could now be knotted together tying down the tendon 40. However, it is preferable to repeat the procedure of FIGS. 8 and 9 with the second suture limb 76 to pass it through the tendon 40 at a third location 88 on an opposite side of the first location 39 as shown in FIG. 10. To ease in knot tying both suture limbs 74 and 76 are preferably pulled out through a single portal such as the auxiliary cannula 78 or other portal through the skin. A knot 90 can then be tied and pushed down to tightly secure the tendon 40 to the humeral head 46 as shown in FIG. 11. By passing the suture limbs 74 and 76 through the tendon 40 at locations 82 and 88 on opposite sides of the first location 39 and defect caused at that location via the passing of the cannula system 48 will be naturally pulled together when the knot 90 is tightened.

Depending upon the extent of the PASTA lesion it may be desirable to place more than one suture anchor 10 beneath the tendon 40. In such case the suture limbs therefrom can be tied together. It would still be preferable to pass them through the tendon at separate locations as illustrated in FIGS. 9 and 10 prior to tying them together, preferably in a mattress pattern. Also, a repair could be fashioned employing one or more knotless suture anchors (not shown) such as disclosed in U.S. Published Application No. 2008/0033486 placed at a location 92 laterally of the tendon 40 and wherein the suture limbs 74 and 76 from the one or more anchors 10 can be passed in a dual row procedure, preferably also employing a mattress pattern. If a lateral anchor is employed, one such method is to put the a pair of present suture anchors 10 anterior and posterior and have one limb 74 from each tied to each other and the other limbs 76 spanned to the lateral anchor (preferably knotless) such that it forms a triangle.

The suture anchor 10 and cannula system 48 may also be used to effect repair of a SLAP (Superior labral tear from Anterior to Posterior) lesion. Typically a much larger traditional cannula (7-8mm) is placed thru the rotator cuff to access the superior labrum for a SLAP repair. The present cannula system is much smaller and also due to its tendency to dilate the tissue rather than be inserted through a large slit would inflict less trauma to the rotator cuff. Such a procedure may be as follows: insert the K wire 38, and then the cannula system 48 in the fashion heretofore described through the rotator interval; drill a hole in the glenoid rim; insert the anchor 10; remove the cannula system 48; pass suture through the labrum using a suture shuttle; and tie knots.

Although described in reference to the optimally narrow suture anchor 10, the cannula system 48 and method of penetrating soft tissue for anchor placement therewith are suitable for other anchors of larger size. For instance they could be
employed with the HEALIX or GRYPHON anchors in sizes 4mm and above available from DePuy Mitek, Inc. of Raynham, MA.

While the invention has been particularly described in connection with specific embodiments thereof, it is to be understood that this is by way of illustration and not of limitation, and that the scope of the appended claims should be construed as broadly as the prior art will permit.

## Claims

1. A trans-soft tissue anchor implantation system comprising:
a positioning wire (38) having a tissue penetrating distal tip;
a cannula system (48) for passage through the soft tissue, the cannula comprising;
an inner cannula (50) having a sharp distal tip (52), an axial lumen (56) therethrough sized to accommodate the positioning wire and a proximal end;
an outer cannula (58) having a distal end (60), a proximal end and an axial lumen (64) therethrough sized to accommodate the inner cannula and coaxially receiving the inner cannula; and
an anchor (10) sized to fit through the outer cannula lumen,
**characterised by** the distal end being tapered wherein to present a gradually increasing profile and wherein the distal tip of the inner cannula extends distally beyond the end of the distal end of the outer cannula, and by the said anchor being a suture anchor.

2. A system according to claim 1 wherein the positioning wire (38) comprises a textured outer surface.

3. A system according to claim 1 and further comprising an engagement between the outer cannula (58) and inner cannula (50) to prevent them from sliding apart.

4. A system according to claim 3 wherein the engagement is a frictional engagement.

5. A system according to claim 3 and further comprising depth indicia (66) on the outer cannula (58).

6. A system according to claim 1 and further comprising a length of suture (30) attached to the suture anchor (10).

## Patentansprüche

1. Weichgewebe durchdringendes Anker-Implantationssystem, umfassend:
einen Positionierdraht (38), aufweisend eine Gewebe durchdringende distale Spitze;
ein Kanülensystem (48) zum Durchgang durch das Weichgewebe, wobei die Kanüle umfasst:
eine innere Kanüle (50), aufweisend eine scharfe distale Spitze (52), ein axiales Lumen (56) dort hindurch, bemessen, um den Positionierdraht und ein proximales Ende aufzunehmen;
eine äußere Kanüle (58), aufweisend ein distales Ende (60), ein proximales Ende und ein axiales Lumen (64) dort hindurch, bemessen, um die innere Kanüle aufzunehmen, das die innere Kanüle koaxial aufnimmt; und
einen Anker (10), bemessen, um durch das Lumen der äußeren Kanüle zu passen,
**dadurch gekennzeichnet, dass** das distale Ende konisch zulaufend ist, um darin ein schrittweise zunehmendes Profil zu präsentieren, und wobei die distale Spitze der inneren Kanüle distal über das Ende des distalen Endes der äußeren Kanüle hinaus verläuft, und **dadurch gekennzeichnet, dass** der Anker ein Fadenanker ist.

2. System nach Anspruch 1, wobei der Positionierdraht (38) eine strukturierte äußere Oberfläche umfasst.

3. System nach Anspruch 1 und weiter umfassend einen Eingriff zwischen der äußeren Kanüle (58) und der inneren Kanüle (50), um deren Auseinandergleiten zu verhindern.

4. System nach Anspruch 3, wobei der Eingriff ein reibschlüssiger Eingriff ist.

5. System nach Anspruch 3 und weiter umfassend Tiefenmarkierungen (66) an der äußeren Kanüle (58).

6. System nach Anspruch 1 und weiter umfassend eine am Fadenanker (10) angebrachte Nahtlänge (30).

## Revendications

1. Système d'implantation d'ancrage à travers des tissus mous, comprenant :
un fil de positionnement (38) comportant une pointe distale de pénétration du tissu ;
un système de canule (48) pour passer à travers les tissus mous, la canule comprenant :
une canule intérieure (50) comportant une pointe distale (52) acérée, une lumière axiale (56) à travers elle, dimensionnée pour recevoir le fil de positionnement, et une extrémité proximale,
une canule extérieure (58) comportant une extrémité distale (60), une extrémité proximale et une lumière axiale (64) à travers elle, dimensionnée pour recevoir la canule intérieure, et recevant coaxialement la canule intérieure ; et
un ancrage (10) dimensionné pour passer à travers la lumière de la canule extérieure,
**caractérisé en ce que** l'extrémité distale est effilée, pour de ce fait présenter un profil progressivement croissant, et dans lequel la pointe distale de la canule intérieure s'étend de façon distale au-delà de l'extrémité de l'extrémité distale de la canule extérieure, et **en ce que** ledit ancrage est un ancrage de suture.

2. Système selon la revendication 1, dans lequel le fil de positionnement (38) comprend une surface extérieure texturée.

3. Système selon la revendication 1, comprenant en outre une entrée en prise entre la canule extérieure (58) et la canule intérieure (50) pour les empêcher de se séparer en coulissant.

4. Système selon la revendication 3, dans lequel l'entrée en prise est une entrée en prise par frottement.

5. Système selon la revendication 3, comprenant en outre des indicateurs (66) de profondeur sur la canule extérieure (58).

6. Système selon la revendication 1, comprenant en outre une longueur de suture (30) fixée à l'ancrage (10) de suture.
